(19) Europäisches Patentamt
European Patent Office
Office européen des brevets

(11) Publication number: 0 135 092
B1

(12) **EUROPEAN PATENT SPECIFICATION**

(45) Date of publication of patent specification: **25.07.90**

(51) Int. Cl.$^5$: **C 12 Q 1/58**, C 12 N 9/06, C 12 Q 1/32

(21) Application number: **84109133.3**

(22) Date of filing: **01.08.84**

(54) **Method for determination of ammonia.**

(30) Priority: **12.08.83 JP 147350/83**

(43) Date of publication of application:
**27.03.85 Bulletin 85/13**

(45) Publication of the grant of the patent:
**25.07.90 Bulletin 90/30**

(84) Designated Contracting States:
**AT CH DE FR GB IT LI NL**

(56) References cited:
**EP-A-0 097 949**
**DE-A-3 240 463**
**FR-A-2 232 760**
**FR-A-2 522 680**

**AGRIC.BIOL.CHEM., vol. 47, no. 1, January 1983, pages 179-182, JP; H.KUSAKABE et al.: "Occurrence of a new enzyme, L-Glutamate oxidase in a wheat bran culture extract of streptomyces sp. X-119-6"**

(73) Proprietor: **Yamasa Shoyu Kabushiki Kaisha**
**10-1, Araoi-Cho 2-Chome**
**Choshi-Shi Chiba-Ken 288 (JP)**

(73) Proprietor: **RIKAGAKU KENKYUSHO**
**2-1 Hirosawa**
**Wako-shi Saitama-ken (JP)**

(72) Inventor: **Murachi, Takashi**
**30, Matsugasaku-Yobikaeshi-Cho Sakyo-Ku**
**Kyoto-Shi Kyoto-Fu (JP)**
Inventor: **Tabata, Masayoshi**
**29-6, Chiyoda-Cho**
**Takatsuki-Shi Osaka-Fu (JP)**

(74) Representative: **Dr. Elisabeth Jung Dr. Jürgen Schirdewahn Dipl.-Ing. Claus Gernhardt**
**P.O. Box 40 14 68 Clemensstrasse 30**
**D-8000 München 40 (DE)**

(56) References cited:
**AGRIC.BIOL.CHEM., vol. 47, no. 6, June 1983, pages 1323-1328, JP; H.KUSAKABE et al.:"Purification and properties of a new enzyme, L-Glutamate oxidase, from streptomyces sp. X-119-6 grown on wheat bran"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 135 092 B1**

**Description**

Background of the Invention

Technical Field

The present invention relates to a novel enzymatic method for determination of ammonia.

The determination of ammonia in the blood is deemed to be an important means for differentiation of hepatic coma, and is required for diagnosis and continuous observation of non-vicarious hepatocirrhosis, fulminant hepatitis and hepatic encephalopathy.

The determination of urea in the blood, on the other hand, is considered to be a significant index in a renal function test. One of the most reliable methods for measuring urea in the blood is to contact a sample with urease to produce ammonia and determine the quantity of the ammonia by the indophenol reaction.

The determination of the concentration of creatinine in serum is also utilized in routine tests as an index of the renal excretory function. A method in which creatinine is catalysed by creatinine deiminase and the quantity of ammonia produced is measured is known as one method for enzymatic determination of creatinine in lieu of the Jaffé reaction heretofore employed.

Thus, by contacting an amino or imino compound (hereinafter referred to as "ammonia-producing substrate") with an enzyme which acts on such a substrate compound to liberate ammonia (hereinafter referred to as "ammonia-producing enzyme") and determining the quantity of the ammonia thus formed, it is possible to measure the concentration of the substrate compound or the enzymatic activity of the ammonia-producing enzyme. The determination of ammonia has the possibility of being applicable to a variety of clinical examinations.

Prior Art

Various methods for determination of ammonia have so far been proposed, among which the enzymatic method, ion exchange method and direct colorimetric method are used widely in the field of clinical examinations.

The enzymatic method involves contacting ammonia with L-glutamate dehydrogenase (GIDH) in the presence of reduced nicotinamide-adenine dinucleotide (NADH) or reduced nicotinamide-adenine dinucleotide phosphate (NADPH) (the abbreviation "NAD(P)H" which will appear hereinafter indicating either "NADH" or "NADPH") and α-ketoglutarate (α-KG) and measuring the decrease in absorbance of NAD(P)H at 340 nm (c.f., for example, J. Lab. Clin. Med. Vol. 66, p. 526 (1965) and Japanese Patent Pub. No. 21995/1982). This method not only requires no treatment for deproteinization of a sample to be measured but also has the advantageous features of high specificity for ammonia and high sensitivity. The enzymatic method, however, has had a limit to its measurement sensitivity since it is also a spectrophotometric method and has further been accompanied by the problem of measurement errors resulting from side reactions of NAD(P)H.

Summary of the Invention

An object of the invention is to solve the above described problems by treating a test sample enzymatically to determine the quantity of ammonia contained therein, using as a signal a measurable value associated with hydrogen peroxide generated.

More specifically, this invention provides a method for determination of ammonia which comprises: (A) contacting a sample to be measured for ammonia content with GIDH in the presence of NAD(P)H and α-KG to produce glutamate in a quantity corresponding to the ammonia content in the sample; (B) catalyzing the resultant mixture of L-glutamate oxidase (GIXD) in the presence of oxygen to produce hydrogen peroxide in a quantity corresponding to the glutamate content in the resultant mixture; (C) measuring the quantity of hydrogen peroxide produced or the production rate thereof; and (D) calculating the ammonia content from the quantity of hydrogen peroxide produced or the production rate thereof thus measured.

The enzyme reactions carried out in the method of the present invention are shown by the following formulae:

$$(1)\ NH_4^+ + NAD(P)H + \alpha\text{-}KG \xrightarrow{GlDH} L\text{-glutamate} + NAD(P)^+$$

$$(2)\ L\text{-glutamate} + H_2O + O_2 \xrightarrow{GlXD} NH_3 + \alpha\text{-}KG + H_2O_2$$

As is set forth hereinabove, the method of the present invention is characterized in that ammonia is converted into L-glutamate by GIDH, and the L-glutamate thus formed is converted into hydrogen peroxide by GIXD, whereupon the quantity of ammonia is determined by measuring the quantity or production rate of the hydrogen peroxide.

By using as a signal a measurable value associated with hydrogen peroxide, it has become possible to apply to the determination of ammonia detection systems ensuring accurate measurements with a simple operation such as the fluorometric method, chemiluminescent method and electrode method in addition to the conventional spectrophotometric method. Heretofore, a method for determination of ammonia by

using as a signal a measurable value associated with hydrogen peroxide has not been known, as far as we are aware, and such a method has been established for the first time by the present invention.

The method of this invention can be carried out by employing a continuous autoanalyzing system using a reactor comprising an immobilized enzyme. In accordance with this invention, the quantity of ammonia can be determined with high sensitivity in a short period of time owing to the continuous autoanalyzing system.

## Brief Description of the Drawings

In the drawings:

Fig. 1 is a diagram showing an example of the organization of a continuous autoanalyzer which is suitable for use in practicing the method of the present invention;

Fig. 2 is a diagram showing the calibration line obtained for ammonia in Example 1 of the present invention;

Fig. 3 is a diagram showing the calibration line obtained for urea in Example 2 of the present invention;

Fig. 4 is a diagram showing the correlation between the values obtained for urea nitrogen in the blood by the method of the present invention in Example 2 and a conventional method;

Fig. 5 is a flow diagram showing a continuous flow-type autoanalyzing system for determination of urea nitrogen in the blood used in Example 3 of the present invention;

Fig. 6 is a diagram showing the correlation between the values obtained for urea nitrogen in the blood by the system shown in Fig. 5 and a conventional system; and

Fig. 7 is a diagram showing the calibration line obtained for creatinine in Example 4 of the present invention.

## Detailed Description of the Invention

### Sample to be analyzed

A sample to be analyzed by the method according to the present invention is an aqueous sample containing ammonia to be measured. The "ammonia" herein includes, in addition to ammonia which is present inherently in the sample, that formed by an ammonia-producing enzyme. The method of the present invention includes not only the detection of ammonia in the sample or the measurement of the content thereof but also, when combined with enzyme reaction systems in which ammonia is formed, the detection or determination of an ammonia-producing substrate or the measurement of the enzymatic activity of an ammonia-producing enzyme.

Examples of such ammonia-producing substrate/ammonia-producing enzyme systems are urea/urease, creatinine/creatinine deiminase, amino acid/amino acid dehydrogenase, amine/amine dehydrogenase, amino acid/ammonia lyase, phosphoramidate and hexose/phosphoramidate-hexose phosphotransferase, adenosine diphosphate (ADP) and carbamoyl phosphate/carbamate kinase, acid amide/amide hydrolase, and a base present in nucleic acid (adenine, cytosine or guanine), nucleoside or nucleotide/deaminase.

Also included in samples to be analyzed by the method of this invention are reaction substrates or reagents employed in single- or multistep reaction systems which can be combined with the enzyme reaction systems mentioned above in which ammonia is formed.

Process steps in the enzyme reaction systems in which ammonia is formed and which are carried out prior to the measurement of ammonia according to the present invention can be controlled by any method or under any conditions suitable for the particular enzyme reaction system selected. In the case where an enzyme reaction system does not interfere with the reaction of GIDH in the first step in the method of this invention, the enzyme reaction can be allowed to proceed in parallel with the first step.

### G1DH Reaction

The reaction in the first step of the method of this invention in which L-glutamate corresponding to ammonia is produced via GIDH is *per se* a known reaction. This GIDH reaction, when applied to the present invention, does not require any special procedure or conditions except for some technical considerations for the subsequent GIXD reaction which must be taken into account. The GIDH reaction is described in known literature and publications, for example, Bergmeyer, H. U.: "Methods of Enzymatic Analysis", 2nd, English Edition, Volume 4, pp. 1802—1806, Academic Press, Inc. (1974) and Japanese Patent Pub. No. 21995/1982.

For GIDH, enzymes of any source or origin such as L-glutamate: $NAD^+$ oxido-reductase (deaminating) (EC 1.4.1.2), L-glutamate: $NAD(P)^+$ oxido-reductase (deaminating) (EC 1.4.1.3), and L-glutamate: $NADP^+$ oxido-reductase (deaminating) (EC 1.4.1.4) can be used.

NADH or NADPH as a coenzyme and α-KG as a reaction substrate are used in excess moles, and the optimum quantity thereof can be easily determined by experimentation depending upon the reaction conditions and the quantity of enzymes employed.

The GIDH reaction is carried out ordinarily at a pH of 7 to 9.5, using as a buffer solution a phosphate, Tris or triethanolamine buffer solution. In the case where the enzyme EC 1.4.1.3 is employed, ADP may be added to activate the enzyme.

GlXD Reaction

The reaction in the second step of the method of this invention in which hydrogen peroxide corresponding to L-glutamate is produced via GlXD is known in the art (c.f. the Agricultural Chemical Society of Japan, Proceedings of the Meeting in 1983, p. 184, 2L-1 (March 10, 1983)). This GlXD reaction is described in more detail in Japanese Patent Laid-Open Pub. No. 42896/1984 (Japanese Patent Appln. No. 145346/1982), which corresponds to European Patent A-Publication No. 0097949.

For GlXD, enzymes having high substrate specificity for L-glutamate are preferably used. Heretofore, GlXD produced from *Streptomyces violascens* (c.f. Japanese Patent Laid-Open Pub. No. 43685/1982) and GlXD produced from *Streptomyces* sp. X-119—6 (FERM P-6560, ATCC 39343) (Agric. Biol. Chem., Vol. 47, No. 6, 1323—1328 (1983)) have been known. The latter enzyme is superior in substrate specificity and stability, particularly pH stability at the optimum pH for GlDH, and is more preferably used in the present invention. Thus, the latter enzyme is designated as GlXD in the following disclosure. The process for producing this enzyme will be set forth in the Reference Example described hereinafter.

The GlXD reaction is carried out ordinarily at a pH of 5 to 10 in a suitable buffer solution.

Measurement of hydrogen peroxide

As methods for measurement of the quantity of hydrogen peroxide produced or the production rate thereof, the chemiluminescence analysis, the electrochemical analysis, the fluorometric analysis, and the spectrophotometric analysis are known in the art (c.f., for example, Journal of Synthetic Chemistry, Japan, Vol. 39, No. 7, pp. 659—666 (1981)).

While any of these analyses is applicable in the present invention, it is preferable for the purpose of the invention to employ a system capable of detecting hydrogen peroxide quickly and with high sensitivity.

Some typical methods for measurement of hydrogen peroxide will now be described below.

In a method in the chemiluminescence analysis, luminol is caused to react with hydrogen peroxide in the presence of potassium ferricyanide, and the quantity of luminescence generated by oxidation is measured. Also known is a method of this type in which luminol is replaced by isoluminol, pyrogallol or bis(2,4,6-trichlorophenyl)oxalate, while potassium ferricyanide is replaced by peroxidase, hematine, hemine or cobalt chloride.

As the electrochemical analysis, a method in which a Clark type hydrogen peroxide electrode is used is generally adopted. A method in which hydrogen peroxide is caused to react with iodine ion in the presence of a catalyst such as peroxidase or molybdate and then the reaction product is measured with an iodine ion electrode can also be used.

In one example of the fluorometric analysis, homovanillic acid is caused to react with hydrogen peroxide in the presence of peroxidase, and the fluorescent intensity of 2,2′-dihydroxy-3,3′-dimethoxybiphenyl-5,5′-diacetic acid formed is measured. It is also possible to replace homovanillic acid with p-hydroxyphenyl acetic acid or a diacetylfluorescein derivative (e.g., diacetylfluorescein or diacetyldichlorofluorescein).

As the spectrophotometric analysis, the peroxidase method and the catalase method are commonly employed.

In the peroxidase method, an oxidizable color former is caused to react with hydrogen peroxide in the presence of peroxidase, and the color formed is determined with a colorimeter. Examples of the color former utilizable in this method are reagents that are used singly such as o-dianisidine, 4-methoxy-1-naphthol, and 2,2′-azino-bis(3-ethylbenzothiazoline)-6-sulfonic acid; a combination of 4-aminoantipyrine with phenol compounds (e.g., phenol, p-chlorophenol and 2,4,6-tribromophenol), aniline compounds (e.g., N,N-dimethylaniline and N,N-diethylaniline) or toluidine compounds (e.g., N,N-diethyl-m-toluidine), and a combination of 3-methyl-2-benzothiazolinone hydrazone with N,N-dimethylaniline.

In the catalase method, hydrogen peroxide is caused to react with an alcohol (e.g., methanol) in the presence of catalase, and the aldehyde formed is introduced into a color forming system in which the absorbance of the colored substance is measured, or the quantity of the aldehyde is determined by utilizing aldehyde dehydrogenase.

All of the above described hydrogen peroxide detection methods are known in the art and can be reduced to practice through a known operation under known conditions.

Determination method and means

Basically, the method of the present invention comprises three steps, namely: the GlDH reaction, the GlXD reaction and the detection of hydrogen peroxide. While these steps may be operated independently by a batchwise procedure, the determination of ammonia can be accomplished more efficiently and in a shorter period of time by employing a continuous autoanalyzing system using a reactor comprising an immobilized enzyme.

Methods for immobilisation of enzymes that are known in the art are broadly classified under the adsorption method, the covalent binding method, the matrix method and the microcapsule method. The immobilization method and immobilizing carrier can be suitably selected by reference to literature (e.g., Chibata, Ichiro (ed.), "Immobilized Enzymes", Kodansha K.K., Japan (March 20, 1975) and Mosbach, K. (ed.), "Method in Enzymology", Vol. XLIV, Immobilized Enzymes, Academic Press, New York (1976)) or publications (e.g., "Biomaterial Science" Series No. 1, Kagaku no Ryoiki (Journal of Japanese Chemistry),

Special No. 134, Nakano-do K.K., Japan (April 20, 1982), pp. 55—67 & 69—79) depending upon the autoanalyzing system employed. With respect to the autoanalyzing system, literature (e.g., Guilbaut, G. G., "HANDBOOK OF ENZYMATIC METHODS OF ANALYSIS", Marcel Dekker, Inc., New York (1976), pp. 445—612 and "Methods in Enzymology" (mentioned *supra*)) may be referred to.

The autoanalyzing system disclosed in Japanese Laid-Open Pub. No. 47484/1983 is suitable for the practice of the present invention. According to this system, it is possible to determine the quantity of ammonia or an ammonia-producing substrate or to measure the activity of an ammonia-producing enzyme in a period of time as short as around ten seconds.

The autoanalyzing system comprises feeding a sample to be analyzed to a column packed with an immobilized enzyme, adding a luminol-potassium ferricyanide, luminescence-inducing agent to the liquid passing through the column and containing hydrogen peroxide formed by the enzyme reaction to induce chemiluminescence, introducing the liquid thus treated into a flow cell, and integrating the quantity of the chemiluminescence from the cell by means of a photon counter or photomultiplier through a luminescence receiver.

Fig. 1 shows the basic structure of the apparatus used in the autoanalyzing system. In Fig. 1, a passage 1 for feeding a sample to be analyzed comprises a passage 2 for feeding a substrate or buffer solution, an inlet 3 for introducing the sample provided in the passage 2, and a column 4 packed with an immobilized enzyme. A passage 5 for feeding a luminescence-inducing agent, on the other hand, comprises a passage 6 for feeding a luminol solution and a passage 7 for feeding potassium ferricyanide provided in parallel, and a spiral tubular mixing coil 8 into which the passage 6 and 7 merge. The quantities of the solutions fed through the passage 2 for a buffer solution, the passage 6 for a luminol solution and the passage 7 for potassium ferricyanide are controlled by a peristaltic pump 9 so that the respective solutions can be fed at predetermined rates.

The passage 1 for feeding a sample to be analyzed and the passage 5 for feeding a luminescence-inducing agent merge and are connected with each other through a means for admixing the two solutions quickly and homogeneously and are further connected to a flow cell 10 which, in turn, is connected to a photomultiplier 11 through a luminescence receiving surface. In accordance with the present invention, the immobilized enzyme column comprises at least immobilized GlDH and GlXD columns connected with each other. In the case of determination of an ammonia-producing substrate by an enzyme reaction in which ammonia is formed, an immobilized ammonia-producing enzyme column is additionally connected thereto. The quantity of each of the immobilized enzymes is determined by experimentation. When a reaction substrate or a coenzyme is required in the reaction in which ammonia is formed, a passage for feeding such a material is further provided as necessary.

## Examples

In order to indicate more fully the nature and utility of this invention, the following specific examples of practice constituting preferred embodiments of the invention and a reference example are set forth, it being understood that these examples are presented as illustrative only and not intended to limit the scope of the invention.

### Example 1
### (Determination of ammonia)

The quantity of ammonia was determined by means of an autoanalyzer having an organization as shown in Fig. 1.

(1) Preparation of immobilized enzyme column

Enzymes were immobilized in accordance with the Weetall method. More particularly, 3 ml of a 2.5% glutaraldehyde solution was added to 200 mg of porous alkylamine glass particles (pore size: 500 Å, and particle size: 80 to 120 mesh, supplied by Pierce Co.), and the resultant mixture was deaerated. The reaction was thereafter allowed to proceed at room temperature under atmospheric pressure for 30 to 60 minutes. The glutaraldehyde solution was then removed, and the remaining glass particles were washed several times with distilled water. To the glass particles was added 1.0 ml of a GlDH solution (8500U, supplied by Toyobo, Japan) in one instance and 2.0 ml of a GlXD solution (130U, see Reference Example set forth hereinafter) in another instance, and the resultant mixtures were respectively caused to react at 4°C overnight. In each case, the reaction solution was removed, and the remaining glass particles were washed at least three times with a suitable buffer solution and then with an aqueous solution of 1M sodium chloride.

The immobilized enzymes thus obtained were packed into respective columns (inner diameter: 1 mm) made of an acrylic resin to prepare immobilized enzyme columns, the GlDH column being 20 mm in length, the GlXD column being 8 mm in length. These columns were connected with each other in the order stated and assembled into the autoanalyzer.

(2) Preparation of reagents

Luminol solution ($7 \times 10^{-4}$M luminol, 0.1M potassium hydroxide, 0.1M boric acid)

Potassium ferricyanide solution ($2 \times 10^{-2}$M)

Substrate buffer solution (containing 1.4 mM α-KG, 0.33 mM NADPH and 0.53 mM EDTA·2Na in 10 mM phosphate buffer solution; pH 7.5)

(3) Determination procedure

1 μl of an aqueous ammonia solution of a known concentration was injected into the autoanalyzer through a microsyringe to measure the quantity of ammonia. The luminol solution, potassium ferricyanide solution and substrate buffer solution were supplied through the peristaltic pump 9 at a rate of 0.6 ml/min.

The relationship between the concentration of ammonia and the quantity of chemiluminescence was as shown by the calibration line illustrated in Fig. 2.

Upon investigation of the optimum concentration of α-KG and NADPH in this determination system, a linear calibration line could be obtained at an α-KG concentration of 5 mg/dl or higher and an NADPH concentration of 15 mg/dl or higher, the ammonia concentration ranging from 0 to 30 mg/dl.

Example 2
(Determination of urea)

An immobilized urease column (0.1 × 2 mm) was prepared as in Example 1 by using 10 mg of urease (ca. 200 U/mg, supplied by Toyobo, Japan), and installed in the autoanalyzer of Example 1 shown in Fig. 1 in the order of immobilized urease, GIDH and GIXD columns.

Subsequently, the quantity of urea was determined by the procedure of Example 1 using a urea solution of a known concentration.

The relationship between the concentration of urea and the quantity of chemiluminescence was as illustrated in Fig. 3.

The quantity of urea nitrogen in the blood was similarly measured using serum. The correlation between the values obtained from the same serum sample by means of a conventional autoanalyzer for measuring the quantity of urea nitrogen in the blood (ureaseindophenol method, Hitachi Model 726) and the autoanalyzer used in accordance with the present invention was found to be satisfactory as is shown in Fig. 4 (n = 63, correlation coefficient r = 0.997, and regression line y = 1.06x − 0.36).

Example 3
(Determination of urea nitrogen in the blood)

A continuous flow-type autoanalyzing system for determination of urea nitrogen shown in the flow diagram of Fig. 5 was prepared. In Fig. 5, a passage 12 for feeding a sample and a passage 13 for feeding a substrate solution are introduced into a 3-inch dialyzer 14 where the substances fed merge. The mixture solution discharged from the dialyzer 14 is applied to an immobilized urease-GIDH (1:2) column 15 (1.5 × 10 mm) and then introduced with dimethylaniline (DMA) solution fed through a passage 16 for DMA into a double mixing coil 17. The mixture solution discharged from the double mixing coil 17 is introduced with 4-aminoantipyrine (4AA) fed through a passage 18 for 4AA into an immobilized GIXD column 19 (1.5 × 10 mm), which is connected to a flow cell 20 (15 mm, 590 nm colorimeter) provided with a recorder 21.

The immobilized enzyme columns were prepared as in Example 1.

Reagents for determination were prepared from the following recipe.

Substrate solution (containing 60 U/dl peroxidase, 5 mg/dl α-KG and 30 mg/dl NADPH in 50 mM phosphate buffer solution: pH 7.5)

4-Aminoantipyrine solution (41.3 mg/dl) (0.05M phosphate buffer solution; pH 5.5)

Dimethylaniline solution (108.8 μl/dl) (0.3N acetic acid solution)

Determination was performed by feeding to the analyzing system serum, physiological saline solution and air at a rate of 0.1 ml/min., 0.8 ml/min. and 0.6 ml/min., respectively, through the passage 12 for a sample solution; the substrate solution and air at a rate of 1.0 ml/min. and 0.8 ml/min., respectively, through the passage 13 for a substrate solution; dimethylaniline solution at a rate of 0.16 ml/min. through the passage 16 therefor; and 4-aminoantipyrine at a rate of 0.16 ml/min. through the passage 18 therefor. The double mixing coil 17 was maintained at a temperature of 48°C.

The correlation between the values thus obtained for urea nitrogen in the blood and those obtained by a conventional autoanalyzer (Hitachi Model 726) in accordance with the urease-indophenol method is shown in Fig. 6. A satisfactory correlation was obtained between the two methods (n = 69, correlations coefficient r = 0.988, and regression line y = 1.1x − 0.5).

Example 4
(Determination of creatinine)

Immobilized creatinine deiminase, GIDH and GIXD were prepared as in Example 1 to form columns of sizes 1.2 × 20 mm, 1.2 × 14 mm and 1.2 × 10 mm which were stacked in order. The stacked columns were installed in the analyzer shown in Fig. 1, and the quantity of creatinine was determined by the procedure of Example 1 using a creatinine solution of a known concentration. As a substrate solution, 10 mM phosphate buffer solution (pH 7.5) containing 0.14 mM α-KG and 0.044 mM NADPH was employed.

The relationship between the concentration of creatinine and the quantity of chemiluminescence was as illustrated in Fig. 7, whereby a straight calibration line could be obtained.

Reference Example
(Production of enzyme)

20 g of wheat bran and 16 ml of water were charged into a 500-ml Erlenmeyer flask and sterilized at 120°C for 30 minutes under pressure. The mixture thus sterilized was inoculated with *Streptomyces* sp. X-119—6 (FERM P-6560), which was cultivated at 28°C for 7 days to prepare an inoculum.

200 g of wheat bran and 160 ml of water were charged respectively into 25 bottles of 5-liter Erlenmeyer flasks, and sterilized at 120°C for 30 minutes under pressure. The resulting mixture was aseptically inoculated with the inoculum prepared in the manner described above, and cultivation was carried out at 28°C for 2 days, and then at 20°C for 2 weeks.

The culture obtained was immersed in 37.5 liters of water and, after filtration, filtered with diatomaceous earth to obtain about 34 liters of a crude enzyme solution. To this crude enzyme solution was added ammonium sulfate to a 50% saturation. The precipitate thus formed was separated, dissolved in 3.9 liters of 0.02M acetate buffer solution (pH 5.5), and heated at 57°C for 30 minutes. After cooling to 5°C or lower, a twofold volume of cold ethanol was added to the resulting solution. The precipitate formed was separated, dissolved in 2 liters of 0.02M phosphate buffer solution (pH 7.4), and dialyzed overnight with the same buffer solution. The precipitate formed during the dialysis was removed while the supernatant was applied to a column (3.5 × 50 cm) packed with DEAE-cellulose equilibrated with 0.02M phosphate buffer solution (pH 7.4) and eluted with 0.02M phosphate buffer solution containing 0.35M sodium chloride. Active fractions thus eluted were collected and dialyzed overnight with 0.05M acetate buffer solution (pH 5.5) containing 0.05M sodium chloride.

The inner dialyzed solution was applied to a column (2 × 10 cm) packed with DEAE-Sepharose® CL-6B equilibrated with the same buffer solution, and eluted with 0.05 to 0.75M sodium chloride solution in accordance with the l near gradient method. Active fractions eluted were collected, concentrated by dialysis, and then subjected to gel filtration through a Sephadex G-200® column (2.5 × 120 cm). Active fractions thus obtained were collected and, after concentration, dialyzed with 0.02M potassium phosphate buffer solution (pH 7.4). The inner dialyzed solution was centrifuged, and the supernatant was subjected to precision filtration and thereafter to freeze drying to obtain 30 mg of a purified GlXD product (specific activity: 55.1 U/mg protein, and yield: 18.4%).

## Claims

1. A method for determination of ammonia which comprises:

(A) contacting a sample to be measured for ammonia content with L-glutamate dehydrogenase in the presence of reduced nicotinamide-adenine dinucleotide or reduced nicotinamide-adenine dinucleotide phosphate and α-ketoglutarate to produce glutamate in a quantity corresponding to the ammonia content in the sample;

(B) catalyzing the resultant mixture by L-glutamate oxidase in the presence of oxygen to produce hydrogen peroxide in a quantity corresponding to the glutamate content in the resultant mixture;

(C) measuring the quantity of hydrogen peroxide produced or the production rate thereof; and

(D) calculating the ammonia content from the quantity of hydrogen peroxide produced or the production rate thereof thus measured.

2. A method as claimed in claim 1, wherein the sample to be measured for ammonia content is an aqueous sample containing ammonia formed by the enzyme reaction between an ammonia-producing enzyme and an ammonia-producing substrate.

3. A method as claimed in claim 1, wherein the L-glutamate oxidase is produced from *Streptomyces* sp. X-119—6 (FERM P-6560, ATCC 39343).

4. A method as claimed in claim 1, wherein the L-glutamate dehydrogenase and the L-glutamate oxidase are in immobilized form, respectively.

## Patentansprüche

1. Ein Verfahren zur Bestimmung von Ammoniak, welches umfaßt:

(A) das Kontaktieren einer auf den Ammoniakgehalt hin zu messenden Probe mit L-Glutamatdehydrogenase in Gegenwart von reduziertem Nikotinamid-Adenindinukleotid oder reduziertem Nikotinamid-Adenindinukleotidphosphat und α-Ketoglutarat zwecks Bildung von Glutamat in einer dem in der Probe vorhandenen Ammoniakgehalt entsprechenden Menge;

(B) das Katalysieren der erhaltenen Mischung mittels L-Glutamatoxidase in Gegenwart von Sauerstoff zwecks Bildung von Wasserstoffperoxid in einer dem in der erhaltenen Mischung vorhandenen Glutamatgehalt entsprechenden Menge;

(C) das Messen der erzeugten Menge an Wasserstoffperoxid oder der Bildungsgeschwindigkeit desselben; und

(D) das Berechnen des Ammoniakgehalts aus der erzeugten Menge an Wasserstoffperoxid oder der so gemessenen Bildungsgeschwindigkeit desselben.

2. Ein Verfahren wie in Anspruch 1 beansprucht, in welchem die auf den Ammoniakgehalt hin zu messende Probe eine wäßrige Probe ist, die durch die Enzymreaktion zwischen einem Ammoniak

produzierenden Enzym und einem Ammoniak produzierenden Substrat gebildetes Ammoniak enthält.

3. Ein Verfahren wie in Anspruch 1 beansprucht, in welchem die L-Glutamatoxidase aus *Streptomyces* sp. X-119—6 (FERM P-6560, ATCC 39343) hergestellt wird.

4. Ein Verfahren wie in Anspruch 1 beansprucht, in welchem die L-Glutamatdehydrogenase und die L-Glutamatoxidase jeweils in immobilisierter Form vorliegen.

**Revendications**

1. Une méthode pour la détermination de l'ammoniaque comprenant les mesures suivantes:

(A) mettre en contact un échantillon, dont le teneur en ammoniaque doit être mesuré, et de la déhydrogenase d'acide L-glutamique en présence de nicotine-amide-adénine-dinucléotide réduit ou de nicotine-amide-adénine-dinucléotide-phosphate réduit et d'alpha-cétoglutarate afin de former du glutamate dans une quantité adéquate au teneur en ammoniaque contenu dans l'échantillon;

(B) catalyser le mélange obtenu au moyen d'oxydase d'acide L-glutamique en présence d'oxygène afin de former du peroxyde d'hydrogène dans une quantité adéquate au teneur en glutamate contenu dans le mélange obtenu;

(C) mesurer le quantité du peroxyde d'hydrogène produite ou son vitesse de formation; et

(D) calculer le teneur en ammoniaque de la quantité de peroxyde d'hydrogène produite ou son vitesse de formation ainsi mesurée.

2. Une méthode selon la revendication 1, dans lequel l'échantillon dont le teneur en ammoniaque doit être mesuré est un échantillon liquide qui contient de l'ammoniaque formé dans la réaction enzymatique entre une enzyme produisant de l'ammoniaque et un substrat produisant de l'ammoniaque.

3. Une méthode selon la revendication 1, dans lequel l'oxydase d'acide L-glutamique est produite du micro-organisme *Streptomyces* sp. X-119—6 (FERM P-6560, ATCC 39343).

4. Une méthode selon la revendication 1, dans lequel la déhydrogenase d'acide L-glutamique et l'oxydase d'acide L-glutamique se trouvent chacune dans une forme immobilisée.

FIG. 1

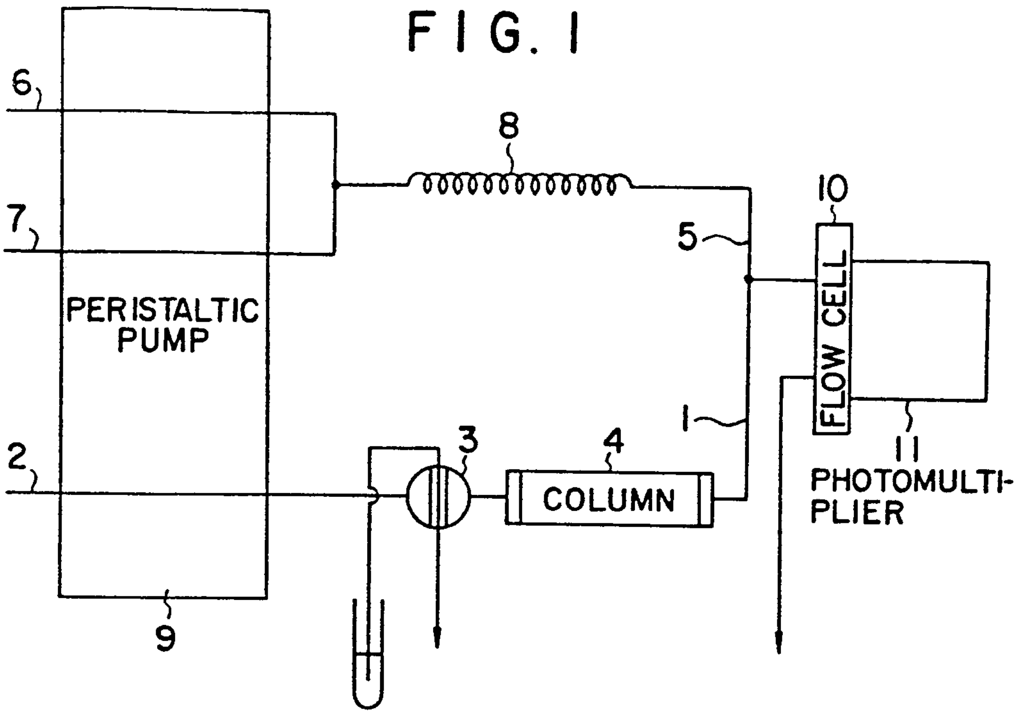


FIG. 2

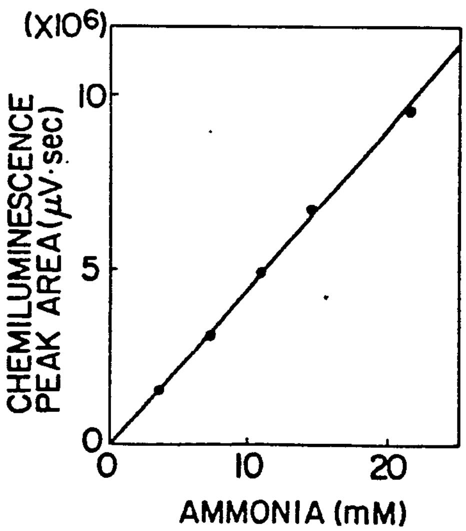


FIG. 3

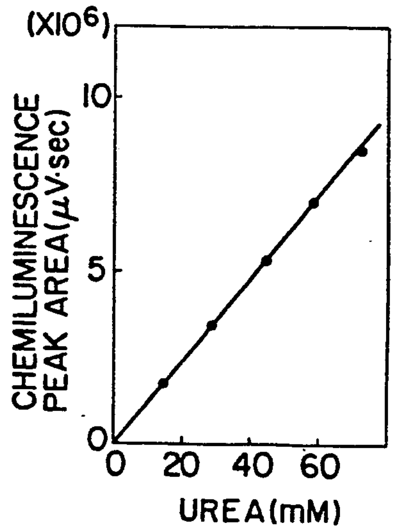

F I G. 4

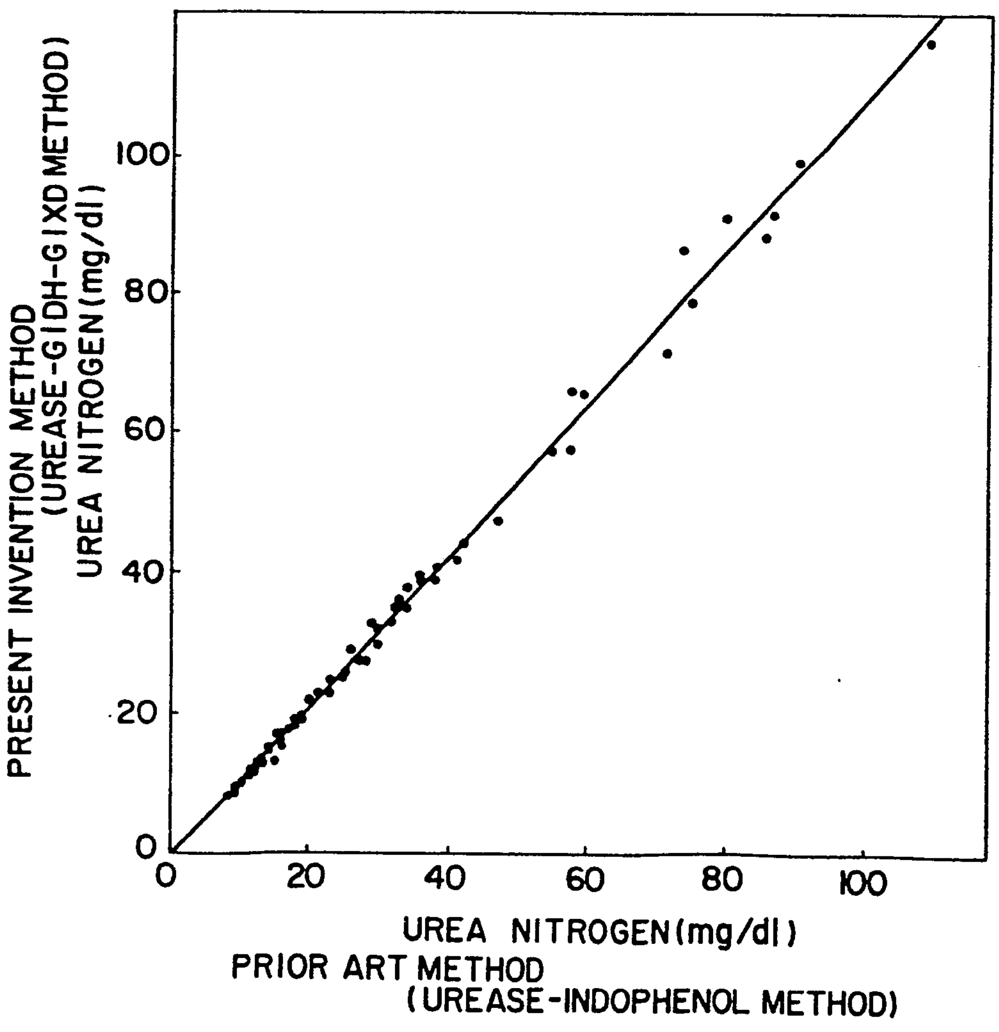
PRESENT INVENTION METHOD
(UREASE-GlDH-GlXD METHOD)
UREA NITROGEN (mg/dl)
0
20
40
60
80
100
0
20
40
60
80
100
UREA NITROGEN (mg/dl)
PRIOR ART METHOD
(UREASE-INDOPHENOL METHOD)

FIG. 5

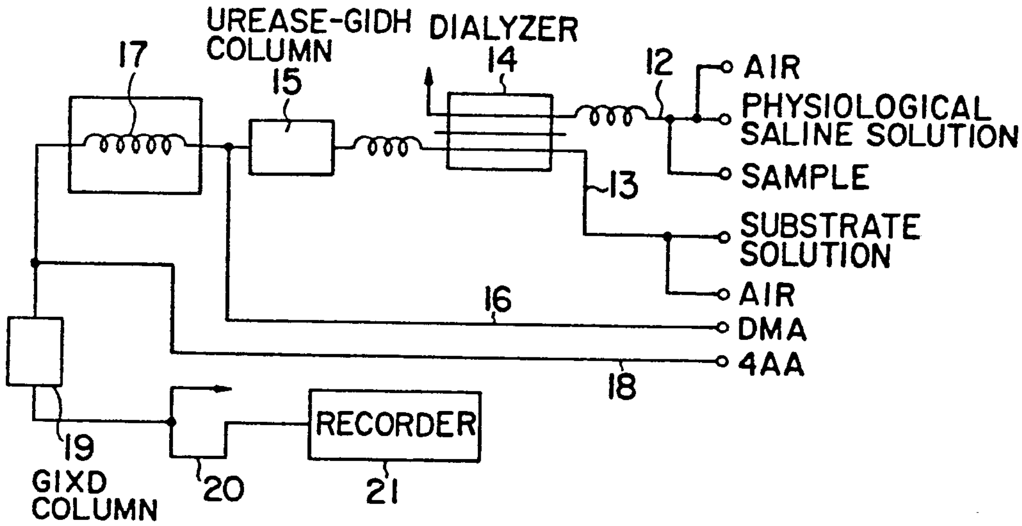
UREASE-GIDH COLUMN
15
DIALYZER
14
17
12
AIR
PHYSIOLOGICAL SALINE SOLUTION
SAMPLE
13
SUBSTRATE SOLUTION
AIR
16
DMA
4AA
18
RECORDER
19
GIXD COLUMN
20
21

FIG. 7

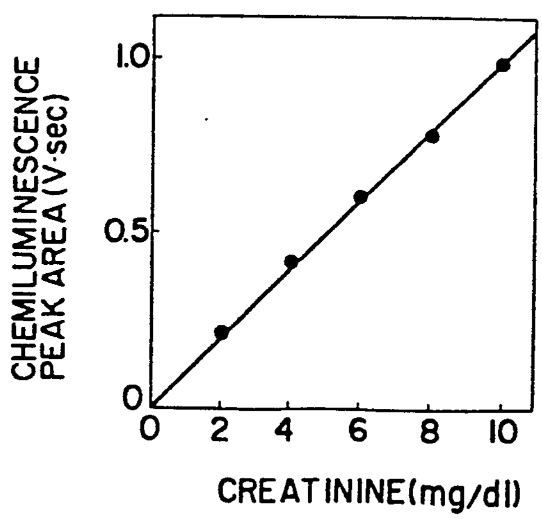
CHEMILUMINESCENCE PEAK AREA (V·sec)
1.0
0.5
0
0
2
4
6
8
10
CREATININE (mg/dl)

F I G. 6

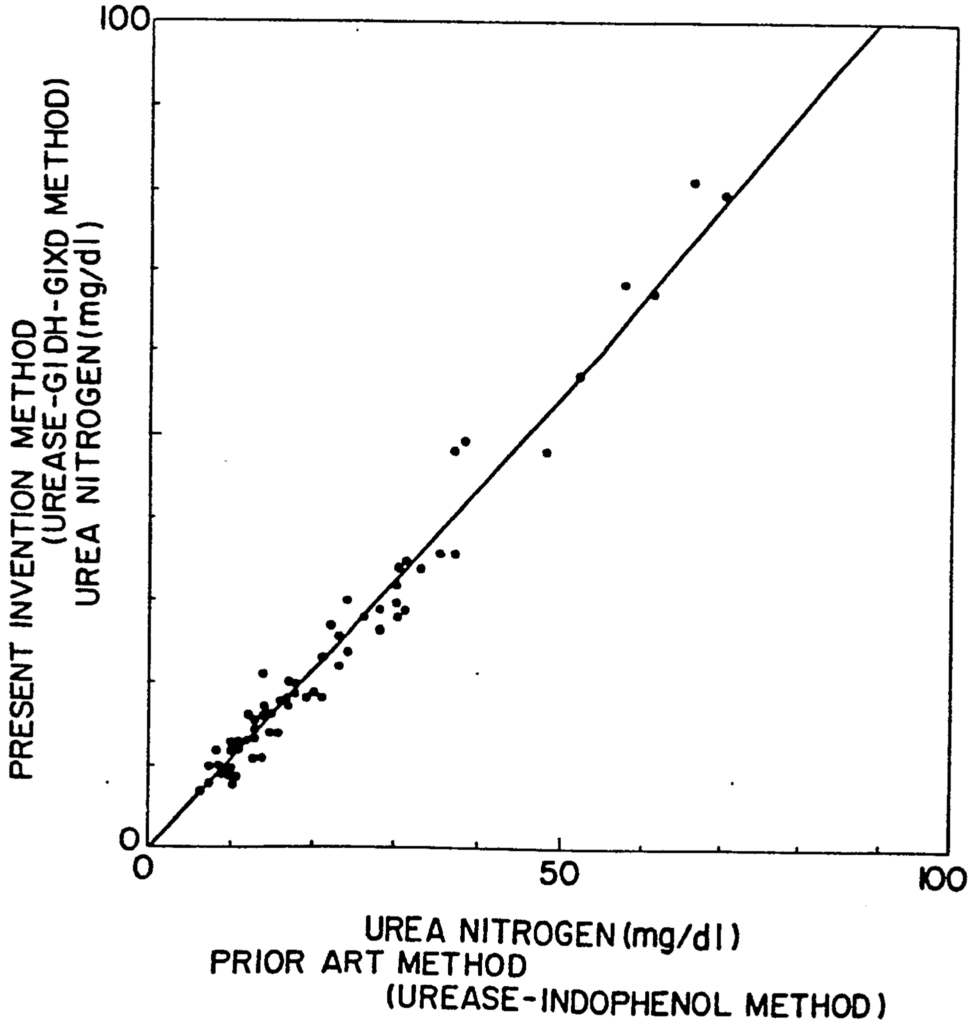
100
0
0
50
100
PRESENT INVENTION METHOD
(UREASE-GlDH-GlXD METHOD)
UREA NITROGEN (mg/dl)
UREA NITROGEN (mg/dl)
PRIOR ART METHOD
(UREASE-INDOPHENOL METHOD)